# EUROPÄISCHE PATENTSCHRIFT

(11) **EP 0 478 937 B1**
(45) Veröffentlichungstag und Bekanntmachung des Hinweises auf die Patenterteilung: **14.12.1994**
(21) Anmeldenummer: 91113763.6
(22) Anmeldetag: 16.08.1991
(51) Int. Cl.: C03C 8/24, C03C 8/06

(54) **Keramischer Werkstoff zum Verblenden von metallischem Zahnersatz**
Ceramic material for covering metallic dental prosthesis
Matériau céramique pour couvrir une prothèse dentale métallique

(30) Priorität: 03.10.1990 DE 4031168
(43) Veröffentlichungstag der Anmeldung: 08.04.1992
(73) Patentinhaber: DEGUSSA AG, 60311 Frankfurt (DE); DUCERA DENTAL-GESELLSCHAFT mbH, 61191 Rosbach (DE)
(72) Erfinder:
(74) Vertreter: Weber, Wolfgang

(56) Entgegenhaltungen:
- FR-A- 955 091
- US-A- 4 349 692
- WORLD PATENTS INDEX LATEST Week 8251, Derwent Publications Ltd., London, GB; AN 82-11217J & SU-A-908 355 (MIROSHNICH) 28. Februar 1982
- PATENT ABSTRACTS OF JAPAN vol. 12, no. 426 (C-542)(3273) 10. November 1988 & JP-A-63 156 036 (KOBE STEEL LTD.) 29. Juni 1988

## Beschreibung

Die Erfindung betrifft einen keramischen Werkstoff zum Verblenden von metallischen Zahnersatz aus niedrigschmelzenden Goldlegierungen oder Titan mit einer Verarbeitungstemperatur von 770° C ± 70° C und einem auf die jeweilige Dentallegierung einstellbaren Wärmeausdehnungskoeffizienten α zwischen 20 und 500° C von 8·10⁻⁶ K⁻¹ bis 9·10⁻⁶ K⁻¹ bzw. 16·10⁻⁶ K⁻¹ bis 17,5·10⁻⁶ K⁻¹.

In der Zahnheilkunde werden seit vielen Jahren nach Art einer Emaillierung auf metallischen Gerüsten (Kronen, Brücken) keramische Schichten als Verblendung vorgenommen, um ein natürliches Aussehen des Zahnersatzes zu erreichen. Dabei werden keramische Pulver als wässriger Schlicker auf das metallische Gerüst aufgetragen und bei hohen Temperaturen gebrannt. Dabei ist es wichtig, daß die Brenntemperatur (Verarbeitungstemperatur) der keramischen Masse wenigstens 100° C unterhalb der Solidustemperatur des Werkstoffes des Metallgerüstes liegt und der Wärmeausdehnungskoeffizient der keramischen Masse im Bereich 20 bis 500° C geringfügig kleiner als der des Metallwerkstoff ist, damit beim Aufbrennen und Abkühlen keine Risse in der Verblendschicht entstehen.

Neuerdings finden gelbe Goldlegierungen mit Goldgehalten zwischen 70 und 85 % Anwendung in der Zahntechnik, die Wärmeausdehnungskoeffizienten zwischen 16 und 17,5·10⁻⁶ K⁻¹ besitzen. Daneben werden Titanwerkstoffe eingesetzt mit Wärmeausdehnungskoeffizienten zwischen 9 und 10·10⁻⁶ K⁻¹. Außerdem besitzen diese Goldlegierungen Solidustemperaturen im Bereich von 870-940° C bzw. bei Titanwerkstoffen ist es wichtig, beim Einbrennen unterhalb der Phasenumwandlungstemperatur von etwa 880° C zu bleiben.

Keramische Massen, die diese Bereiche des Wärmeausdehnungskoeffizienten abdecken und gleichzeitig eine Verarbeitungstemperatur im Bereich von etwa 700-840° C besitzen, sind als Verblendmaterialien für metallischen Zahnersatz bisher nicht bekannt geworden.

Aus der FR-A-955091 sind Spezialgläser für Geräte, insbesondere für Kathodenstrahlröhren bekannt, die aus 53 bis 75 % SiO₂, 3 bis 15 % Al₂O₃, 0,1 bis 13 % K₂O. 0,1 bis 17 % Na₂O, 0,5 bis 2 % Li₂O , 0 bis 28 % BaO und 0,5 bis 2,5 % Fluor bestehen. Solche Spezialgläser sind nicht für das Verblenden von metallischem Zahnersatz einsetzbar, auch wenn sie einen Wärmeausdehnungskoeffizienten von 8 bis 10·10⁻⁶ K⁻¹ besitzen.

In der US-A 4,349,692 wird ein Glas für elektrische Einschmelzdrähte beschrieben, das aus 63 bis 68 % SiO₂, 3 bis 6 % Al₂O₃, 8 bis 9 % K₂ 0,5 bis 6 % Na₂O, 0,5 bis 1,5 % Li₂O, 2 bis 4 % BaO, 5 bis 7 % SrO, 2 bis 4 % CaO, 0,5 bis 1,5 % MgO, 0,5 bis 1,5 % TiO₂ und 0,5 bis 1,5 % B₂O₃ besteht. Auch solche Gläser sind nicht als Verblendmaterial für metallischen Zahnersatz einsetzbar.

Aus den Patent Abstracts of Japan, vol. 12, no. 246 (C-542)(3271) vom 10.11.1988 ist ein Verblendmaterial für Zahnersatz aus Titan bekannt, das 45 bis 65 % SiO₂ 2 bis 10 % B₂O₃, 10 bis 20 % Al₂O₃, insgesamt 7 bis 20 % Na₂O, K₂O und Li₂O und 1 bis 10 % CaO, MgO und BaO enthält. Diese Verblendmaterialien haben sich in der Praxis allerdings nicht bewährt.

Es war daher Aufgabe der vorliegenden Erfindung einen keramischen Werkstoff zum Verblenden von metallischem Zahnersatz aus niedrigschmelzenden Goldlegierungen und Titan zu entwickeln, der eine Verarbeitungstemperatur von 770° C ± 70° C und einen Wärmeausdehnungskoeffizienten zwischen 20 und 500° C aufweist, der auf Werte zwischen 8·10⁻⁶ und 9·10⁻⁶ K⁻¹ bzw. 16·10⁻⁶ und 17,5·10⁻⁶ K⁻¹ einstellbar ist.

Diese Aufgabe wird für Goldlegierungen mit Wärmeausdehnungskoeffizienten zwischen 16 und 17,5·10⁻⁶ K⁻¹ von keramischen Werkstoffen mit folgender Zusammensetzung gelöst: 60-68 Gew.% SiO₂, 10-15 Gew.% Al₂O₃, 0,7-1,5 Gew.% B₂O₃, 0-0,5 Gew.% Sb₂O₃, 0-0,5 Gew.% BaO, 0,1-0,5 Gew.% CaO, 9-12 Gew.% K₂O, 9-11 Gew.% Na₂O, 0,8-1,4 Gew.% Li₂O und 0,2-0,4 Gew.% F₂.

Besonders bewährt haben sich folgende Zusammensetzungen: 62-65 Gew.% SiO₂, 12-15 Gew.% Al₂O₃, 0,8-1,2 Gew.% B₂O₃, 0-0,2 Gew.% Sb₂O₃, 0-0,4 Gew.% CeO₂, 0-0,1 Gew.% BaO, 0,2-0,4 Gew.% CaO, 9-11 Gew.% K₂O, 9-11 Gew.% Na₂O, 0,8-1,2 Gew.% Li₂O und 0,2-0,4 Gew.% F₂.

Für Titan und Titanlegierungen mit Wärmeausdehnungskoeffizienten von 9-10·10⁻⁶ K⁻¹ verwendet man keramische Werkstoffe mit folgenden Zusammensetzungen: 68-75 Gew.% SiO₂, 5-8 Gew.% Al₂O₃, 2-2,5 Gew.% B₂O₃, 0,3-0,9 Gew.% Sb₂O₃, 0-0,2 Gew.% CeO₂, 1,5-2,5 Gew.% BaO, 0-0,3 Gew.% CaO, 7-11 Gew.% K₂O, 6-10 Gew.% Na₂O, 0,55-0,75 Gew.% Li₂O und 0,8-1,0 Gew.% F₂.

Besonders bewährt haben sich Zusammensetzungen mit 70-72 Gew.% SiO₂, 5-7 Gew.% Al₂O₃, 2,1-2,4 Gew.% B₂O₃, 0,4-0,6 Gew.% Sb₂O₃, 1,8-2,2 Gew.% BaO, 0-0,1 Gew.% CaO, 7-9 Gew.% K₂O, 7-9 Gew.% Na₂O, 0,55-0,75 Gew.% Li₂O und 0,8-1,0 Gew.% F₂.

Diese keramische Werkstoffe besitzen alle eine Verarbeitungstemperatur im Bereich zwischen 700 und 840° C.

Folgende Beispiele sollen die Erfindung näher erläutern:
1. Eine gelbe Dentallegierung aus 77 % Gold, 9 % Silber, 2 % Palladium, 4,3 % Platin, 4,5 % Kupfer, 2 % Indium und 1,2 % Zink mit einer Solidustemperatur von 900° C und einem Wärmeausdehnungskoeffizienten von 16,5·10⁻⁶ K⁻¹ wurde mit einer keramischen Masse der Zusammensetzung 63,2 % SiO₂, 12,8 % Al₂O₃, 0,8 % B₂O₃, 0,2 % Sb₂O₃, 0,2 % CeO₂, 0,1 % BaO, 0,3 % CaO, 10,6 % K₂O, 10,4 % Na₂O, 1,1 % Li₂O und 0,3 % Fluorid bei 770° C verblendet.
   Die keramische Masse besaß einen dilatometrischen Entweichungspunkt von 550° C, einen Glaspunkt von 460° C und einen Wärmeausdehnungskoeffizienten von 16,2·10⁻⁶K⁻¹.
   Nach dem Aufbrennen hatte die keramische Masse eine sehr gute Oberflächenstruktur, die über dem gewohnten Niveau der bekannten Keramiken lag, während die Transparenz mit diesen Massen vergleichbar war.
   Der Schertest nach Entwurf DIN 13927 beträgt 34N/mm². Dieser Wert liegt im Vergleich zu anderen Aufbrennlegierungen und normalen Metallkeramikmassen im mittleren Bereich.
   Die Verbundprüfung (qualitativer Hafttest) nach ISO 9693.2 und Entwurf DIN 13927 wird überragend bestanden.
   Die Hydrolysebeständigkeit - 16 Stunden kochen in 4%iger Essigsäure nach ISO 9693.2 und DIN 13925 bzw. Entwurf DIN 13927 - ergab praktisch keinen wägbaren Masseverlust. Der Glanzzustand der Probekörper ist praktisch unverändert.
   Die Biegefestigkeit nach DIN 13925 und ISO 9693.2 liegt bei 75 N/mm² und somit ca. 50 % über der nach DIN und ISO geforderten Mindestbiegefestigkeit von 50 N/mm².
2. Ein Titan-Brückengerüst mit einem Wärmeausdehnungskoeffizienten von 9,6 10⁻⁶K⁻¹ wurde mit einer keramischen Masse der Zusammensetzung 72,5 Gew.% SiO₂, 4,5 Gew.% Al₂O₃, 2,5 Gew.% B₂O₃, 0,3 Gew.% Sb₂O₃, 2,2 Gew.% BaO, 7,5 Gew.% K₂O, 9,0 Gew.% Na₂O, 0,7 Gew.% Li₂O und 0,8 Gew.% Fluorid bei 720° C verblendet.
   Die keramische Masse besitzt einen dilatometrischen Erweichungspunkt von 570° C, einen Glaspunkt von 480° C und einen Wärmeausdehnungskoeffizienten von 8,3 x 10⁻⁶K⁻¹.
   Nach Einbrennen war die Transparenz sowie auch die Oberflächenstruktur der keramischen Massen bestechend gut und lagen über dem gewohnten Niveau der normalen Metallkeramikmassen. Der Schertest nach Entwurf DIN 13927 betrug im Durchschnitt 30 N/mm².
   Die Verbundprüfung (qualitativer Hafttest) nach ISO 9693.2 und Entwurf DIN 13927 wurde überragend bestanden. Die Hydrolysebeständigkeit, 16 Stunden kochen in 4 %iger Essigsäure nach ISO 9693.2 und DIN 13925 bzw. Entwurf DIN 13927, ergab praktisch keinen wägbaren Masseverlust. Der Glanzzustand der Probekörper war praktisch unverändert. Die Biegefestigkeit nach DIN 13925 und ISO 9693.2 lag bei 85 N/mm² und somit ca. 70 % über der nach DIN und ISO geforderten Mindestbiegefestigkeit von 50 N/mm².

## Patentansprüche

1. Keramischer Werkstoff zum Verblenden von metallischem Zahnersatz aus niedrigschmelzenden Goldlegierungen mit einem Wärmeausdehnungskoeffizienten zwischen 16·10⁻⁶ und 17,5·10⁻⁶ K⁻¹ und einer Verarbeitungstemperatur von 770 ± 70° C.
**dadurch gekennzeichnet**,
daß er sich zusammensetzt aus: 60 bis 68 Gew.% SiO₂, 10 bis 15 Gew.% Al₂O₃, 0,7 bis 1,5 Gew.% B₂O₃, 0 bis 0,5 Gew.% Sb₂O₃, 0 bis 0,5 Gew.% CeO₂, 0 bis 0,5 Gew.% BaO, 0,1 bis 0,5 Gew.% CaO, 9 bis 12 Gew.% K₂O, 9 bis 11 Gew.% Na₂O, 0,8 bis 1,4 Gew.% Li₂O und 0,2 bis 0,4 Gew.% F₂.

2. Keramischer Werkstoff nach Anspruch 1,
**dadurch gekennzeichnet**,
daß er sich zusammensetzt aus: 62 bis 65 Gew.% SiO₂, 12 bis 15 Gew.% Al₂O₃, 0,8 bis 1,2 B₂O₃, 0 bis 0,2 Gew.% Sb₂O₃, 0 bis 0,4 Gew.% CeO₂, 0 bis 0,1 Gew.% BaO, 0,2 bis 0,4 CaO, 9 bis 11 Gew.% K₂O, 9 bis 11 Gew.% Na₂O, 0,8 bis 1,2 Gew.% Li₂O und 0,2 bis 0,4 Gew.% F₂.

3. Keramischer Werkstoff zum Verblenden Von metallischem Zahnersatz aus Titan und Titanlegierungen mit einem Wärmeausdehnungskoeffizienten zwischen 8·10⁻⁶ und 9·10⁻⁶ K⁻¹ und einer Verarbeitungstemperatur von 770 ± 70° C.
**dadurch gekennzeichnet**,
daß es sich zusammensetzt aus: 68 bis 75 Gew.% SiO₂, 4 bis 8 Gew.% Al₂O₃, 2 bis 2,5 Gew.% B₂O₃, 0,3 bis 0,9 Gew.% Sb₂O₃, 0 bis 0,2 Gew.% CeO₂, 1,5 bis 2,5 Gew.% BaO, 0 bis 0,3 Gew.% CaO, 7 bis 11 Gew.% K₂O, 6 bis 10 Gew.% Na₂O, 0,55 bis 0,75 Gew.% Li₂O und 0,8 bis 1,0 Gew.% F₂.

4. Keramischer Werkstoff nach Anspruch 3,
**dadurch gekennzeichnet**,
daß er sich zusammensetzt aus: 70 bis 74 Gew.% SiO₂, 4 bis 7 Gew.% Al₂O₃, 2,1 bis 2,4 Gew.% B₂O₃, 0,4 bis 0,6 Gew.% Sb₂O₃, 1,8 bis 2,2 Gew.% BaO, 0 bis 0,1 Gew.% CaO, 7 bis 9 Gew.% K₂O, 7 bis 9 Gew.% Na₂O, 0,55 bis 0,75 Gew.% Li₂O und 0,8 bis 1,0 Gew.% F₂.

## Claims

1. Ceramic material for facing metal dentures made from low-melting gold alloys with a coefficient of thermal expansion between 16 x 10⁻⁶ and 17.5 x 10⁻⁶ K⁻¹and a processing temperature of 770 ± 70°C
characterised in that
it consists of: 60 to 68 wt.% SiO₂, 10 to 15 wt.% Al₂O₃, 0.7 to 1.5 wt.% B₂O₃, 0 to 0.5 wt.% Sb₂O₃, 0 to 0.5 wt.% CeO₂, 0 to 0.5 wt.% BaO, 0.1 to 0.5 wt% CaO, 9 to 12 wt.% K₂O, 9 to 11 wt.% Na₂O, 0.8 to 1.4 wt.% Li₂O and 0.2 to 0.4 wt.% F₂.

2. Ceramic material according to Claim 1,
characterised in that
it consists of: 62 to 65 wt.% SiO₂, 12 to 15 wt.% Al₂O₃, 0.8 to 1.2 wt.% B₂O₃, 0 to 0.2 wt.% Sb₂O₃, 0 to 0.4 wt.% CeO₂, 0 to 0.1 wt.% BaO, 0.2 to 0.4 wt.% CaO, 9 to 11 wt.% K₂O, 9 to 11 wt.% Na₂O, 0.8 to 1.2 wt.% Li₂O and 0.2 to 0.4 wt.% F₂.

3. Ceramic material for facing metal dentures made from titanium and titanium alloys with a coefficient of thermal expansion between 8 x 10⁻⁶ and 9 x 10⁻⁶ K⁻¹ and a processing temperature of 770 ± 70°C
characterised in that
it consists of: 68 to 75 wt.% SiO₂, 4 to 8 wt.% Al₂O₃, 2 to 2.5 wt.% B₂O₃, 0.3 to 0.9 wt.% Sb₂O₃, 0 to 0.2 wt.% CeO₂, 1.5 to 2.5 wt.% BaO, 0 to 0.3 wt.% CaO, 7 to 11 wt.% K₂O, 6 to 10 wt.% Na₂O, 0.55 to 0.75 wt.% Li₂O and 0.8 to 1.0 wt.% F₂.

4. Ceramic material according to Claim 3,
characterised in that
it consists of: 70 to 74 wt.% SiO₂, 4 to 7 wt.% Al₂O₃, 2.1 to 2.4 wt.% B₂O₃, 0.4 to 0.6 wt.% Sb₂O₃, 1.8 to 2.2 wt.% BaO, 0 to 0.1 wt.% CaO, 7 to 9 wt.% K₂O, 7 to 9 wt.% Na₂O, 0.55 to 0.75 wt.% Li₂O and 0.8 to 1.0 wt.% F₂.

## Revendications

1. Matière céramique destinée à recouvrir une prothèse dentaire métallique composée d'alliages en or à bas point de fusion, ayant un coefficient de dilatation thermique compris entre 16.10⁻⁶ et 17,5.10⁻⁶K⁻¹ et une température de traitement de 770 ± 70°C, caractérisée en ce qu'elle est composée de :
60 à 68 % en poids de SIO₂
10 à 15 % en poids d'Al₂O₃
0,7 à 1,5 % en poids de B₂O₃
0 à 0,5 % en poids de Sb₂O₃
0 à 0,5 % en poids de CeO₂
0 à 0,5 % en poids de BaO
0,1 à 0,5 % en poids de CaO
9 à 12 % en poids de K₂O
9 à 11 % en poids de Na₂O
0,8 à 1,4 % en poids de Li₂O
0,2 à 0,4 % en poids de F₂

2. Matière céramique selon la revendication 1, caractérisée en ce qu'elle est composée de :
62 à 65% en poids de SiO₂
12 à 15 % en poids d'Al₂O₃
0,8 à 1,2 % en poids de B₂O₃
0 à 0,2 % en poids de Sb₂O₃
0 à 0,4 % en poids de CeO₂
0 à 0,1 % en poids de BaO
0,2 à 0,4 % en poids de CaO
9 à 11 % en poids de K₂O
9 à 11 % en poids de Na₂O
0,8 à 12 % en poids de Li₂O
0,2 à 0,4 % en poids de F₂

3. Matière céramique destinée à recouvrir une prothèse dentaire métallique composée de titane et d'alliages de titane, ayant un coefficient de dilatation thermique compris entre 8.10⁻⁶ et 9.10⁻⁶K⁻¹ et une température de traitement de 770 ± 70°C, caractérisée en ce qu'elle est composée de
68 à 75 % en poids de SiO₂
4 à 8 % en poids d'Al₂O₃
2 à 2,5 % en poids de B₂O₃
0,3 à 0,9 % en poids de Sb₂O₃
0 à 0,2 % en poids de CeO₂
1,5 à 2,5 % en poids de BaO
0 à 0,3 % en poids de CaO
7 à 11 % en poids de K₂O
6 à 10 % en poids de Na₂O
0,55 à 0,75 % en poids de Li₂O
0,8 à 1,0 % en poids de F₂

4. Matière céramique selon la revendication 3, caractérisée en ce qu'elle est composée de :
70 à 74 % en poids de SiO₂
4 à 7 % en poids d'Al₂O₃
2,1 à 2,4 % en poids de B₂O₃
0,4 à 0,6 % en poids de Sb₂O₃
1,8 à 2,2 % en poids de BaO
0 à 0,1 % en poids de CaO
7 à 9 % en poids de K₂O
7 à 9 % en poids de Na₂O
0,55 à 0,75 % en poids de Li₂O
0,8 à 1 % en poids de F₂
